(19) **Europäisches Patentamt / European Patent Office / Office européen des brevets**

(11) **EP 2 486 953 B1**

(12) **FASCICULE DE BREVET EUROPEEN**

(45) Date de publication et mention de la délivrance du brevet:
**01.06.2016 Bulletin 2016/22**

(51) Int Cl.:
*A61N 1/372* (2006.01)   *G06F 1/06* (2006.01)
*G06F 1/12* (2006.01)

(21) Numéro de dépôt: **12153636.1**

(22) Date de dépôt: **02.02.2012**

(54) **Procédé de quantification de la désynchronisation entre les horloges de deux implants actifs de type HBC**

Verfahren zur Quantifizierung der Desynchronisation zwischen den Uhren von zwei aktiven Implantaten vom Typ HBC

Method for quantifying the desynchronisation between the clocks of two active HBC implants

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **09.02.2011 FR 1151021**

(43) Date de publication de la demande:
**15.08.2012 Bulletin 2012/33**

(73) Titulaire: **Sorin CRM SAS**
**92140 Clamart Cedex (FR)**

(72) Inventeurs:
• **Dal Molin, Renzo**
**92320 Châtillon (FR)**
• **Ghildiyal, Ashutosh**
**92140 Clamart (FR)**

(74) Mandataire: **Dupuis-Latour, Dominique et al**
**Bardehle Pagenberg**
**10, boulevard Haussmann**
**75009 Paris (FR)**

(56) Documents cités:
WO-A1-2011/093916   US-A1- 2005 197 680
US-A1- 2007 088 394   US-A1- 2007 255 330
US-A1- 2009 030 484

**Description**

L'invention concerne, de façon générale, le domaine des "dispositifs médicaux actifs" tels que définis par la directive 93/42/CE du 14 juin 1993 du Conseil des communautés européennes, et notamment les "dispositifs médicaux implantables actifs" tels que définis par la directive du Conseil 90/385/CEE du 20 juin 1990.

Cette définition inclut en particulier les appareils chargés de surveiller l'activité cardiaque et de générer des impulsions de stimulation, de resynchronisation, de défibrillation et/ou de cardioversion en cas de trouble du rythme détecté par l'appareil. Elle inclut aussi les appareils neurologiques, les implants cochléaires, etc., ainsi que les dispositifs de mesure de pH ou encore d'impédance intracorporelle (telle que mesure d'impédance transpulmonaire ou d'impédance intracardiaque).

L'invention concerne plus particulièrement ceux de ces dispositifs qui mettent en oeuvre des capsules autonomes implantées et dépourvues de toute liaison physique à un dispositif principal implanté (tel qu'un boîtier de générateur d'impulsions de stimulation) ou non implanté (périphérique externe tel que programmateur ou dispositif de *monitoring* pour le suivi à distance du patient). La communication, conduite par les tissus interstitiels du corps, est alors du type dit "HBC" (*Human Body Communication,* communication par voie intracorporelle).

Ces capsules autonomes sont dénommées pour cette raison "capsules *leadless*", pour les distinguer des électrodes ou des capteurs disposés à l'extrémité distale d'une sonde (*lead*), cette sonde étant parcourue sur toute sa longueur par un ou plusieurs conducteurs reliant par voie galvanique l'électrode ou le capteur à un générateur connecté à l'extrémité opposée, proximale, de la sonde.

De telles capsules *leadless* sont par exemple décrites dans les US 2007/0088397 A1 et WO 2007/047681 A2 (Nanostim, Inc.) ou encore dans le US 2006/0136004 A1 (EBR Systems, Inc.).

Ces capsules *leadless* peuvent être notamment des capsules épicardiques, fixées à la paroi extérieure du coeur, ou bien des capsules endocavitaires, fixées à la paroi intérieure d'une cavité ventriculaire ou auriculaire. Leur fixation à la paroi cardiaque se fait habituellement au moyen d'une vis d'ancrage hélicoïdale saillante, prolongeant axialement le corps de la capsule et destinée à pénétrer dans le tissu cardiaque par vissage au site d'implantation.

Une telle capsule comprend des circuits de détection/stimulation pour recueillir des potentiels de dépolarisation du myocarde et/ou pour appliquer des impulsions de stimulation au site où est implantée la capsule. La capsule porte alors une électrode appropriée, qui peut être notamment constituée par une partie active de la vis d'ancrage ou par des électrodes sur le boîtier. Elle peut également incorporer un ou plusieurs capteurs permettant de mesurer localement la valeur d'un paramètre tel que le niveau d'oxygène dans le sang, la pression cardiaque endocavitaire, l'accélération de la paroi cardiaque, l'accélération du patient come indicateur de l'activité etc.

L'invention n'est toutefois pas limitée à un type particulier de capsule, et elle est applicable indifféremment à tout type de capsule *leadless,* quelle que soit sa destination fonctionnelle.

Bien entendu, pour permettre l'échange de données à distance, les capsules *leadless* incorporent des moyens émetteurs/récepteurs de communication sans fil.

Plusieurs techniques ont été proposées pour assurer la communication sans fil entre ces capsules autonomes et/ou un dispositif distant destiné à centraliser les informations recueillies par les capsules et à leur envoyer si nécessaire des commandes appropriées (ce dispositif peut être notamment un stimulateur, resynchroniseur ou défibrillateur implanté, un défibrillateur sous-cutané, ou un enregistreur longue durée).

Ainsi, le US 2006/0136004 A1 propose de transmettre les données par des ondes acoustiques se propageant à l'intérieur du corps. Cette technique est efficace et sans danger ; elle présente toutefois l'inconvénient de nécessiter une puissance d'émission relativement élevée compte tenu de l'atténuation des ondes acoustiques dans le corps, et ne permet qu'un débit de données relativement faible.

Le US 5 411 535 A propose une autre technique, basée sur l'utilisation d'ondes radiofréquence (RF). Ici encore, une puissance d'émission relativement importante est nécessaire, et l'atténuation de ces ondes par les tissus intracorporels est un obstacle important à leur propagation.

Une autre technique encore a été proposée par le US 4 987 897 A, mais il s'agit d'un échange de données avec un dispositif externe (programmateur), par voie transcutanée et non plus intracorporelle. Cette transmission est assurée à courte distance entre, d'une part, le boîtier d'un stimulateur implanté dans une poche sous-cutanée et, d'autre part, un programmateur externe posé contre la peau, à proximité du générateur. Les courants circulent donc au travers de la peau dans une région très éloignée des endroits sensibles, notamment à distance du myocarde, ce qui évite tout risque de perturbation des ondes de dépolarisation naturelle ou stimulée de ce dernier.

Le US 2007/0088397 A1 propose par ailleurs d'utiliser les impulsions de stimulation produites par une capsule comme véhicule pour la transmission de données préalablement recueillies ou élaborées par la capsule. Pour cela, l'impulsion, au lieu de présenter une variation monotone de tension, est interrompue de manière contrôlée pendant des durées très brèves de manière à créer dans le profil de l'impulsion de très étroits créneaux dont la succession correspond à un codage binaire de l'information à transmettre.

Le traitement des signaux échangés entre les capsules et/ou le dispositif principal implanté (typiquement le stimulateur ou défibrillateur) ou, de façon plus générale, entre les divers dispositifs autonomes implantés dans le corps du patient, implique de synchroniser les circuits de traitement respectifs de ces dispositifs, ou à tout le moins d'en déterminer le degré de désynchronisation. S'il est connu, ce dernier paramètre pourra être pris en compte dans les différents calculs, ou servir à appliquer une correction temporelle appropriée à la datation des signaux reçus ou émis. On pourra ainsi opérer réaliser une "synchronisation logicielle" ne nécessitant pas d'appliquer une action de correction ou de réglage sur les circuits des horloges des dispositifs.

Dans la mesure où les divers dispositifs de l'ensemble sont physiquement indépendants, chacun possède son horloge propre qui assure le pilotage des divers circuits, notamment les circuits de traitement numérique des signaux.

Ces horloges, indépendantes, présentent un écart de fréquence qui, même minime, va introduire au cours du temps une désynchronisation des dispositifs entre eux, qu'il y aura lieu d'identifier et de compenser. Dans un domaine voisin, le US2009/030484 A1 propose de synchroniser le séquencement de deux audioprothèses séparées, disposées sur les deux oreilles d'un patient, afin de réduire les interférences mutuelles susceptibles d'être générées. Ces audioprothèses sont munies d'horloges propres, et de moyens d'échange de données de resynchronisation permettant de recaler entre elles les deux horloges.

Le problème du fonctionnement synchrone d'implants *leadless* est également abordé par le US2007/0255330 A1, qui enseigne de mesurer l'intervalle de temps entre la détection en deux régions différentes du patient d'un même événement physiologique, ou encore par le US 205/0197680 A1 qui prévoit d'agir sur une boucle à verrouillage de phase pour le contrôle fin de la fréquence de l'horloge interne.

En tout état de cause, dans le cas d'implants, les dérives de fréquence sont accrues par le fait que, pour éviter une consommation excessive, les implants utilisent des horloges à relativement basse fréquence (typiquement de l'ordre de 32 kHz), avec de ce fait une résolution temporelle plus faible (environ 30 μs) ne permettant pas une évaluation fine des écarts de synchronisation. Cet aspect est notamment évoqué, en tant que tel, par le US 2007/0088394 A1, mais en dehors du contexte de la synchronisation entre les implants.

Le WO 2011/093916 aussi divulgue un procédé de synchroniser deux dispositifs médicaux implantables correspondant au préambule de la revendication 1.

En effet, quelle que soit la technique mise en oeuvre, le traitement du signal HBC recueilli ou émis par une capsule leadless nécessite une énergie non négligeable par rapport aux ressources énergétiques dont dispose cette capsule. Compte tenu de son caractère autonome, la capsule ne peut en effet faire appel qu'à ses ressources propres telles qu'un circuit de récupération d'énergie (par le mouvement de la capsule) et/ou une petite batterie intégrée.

La gestion de l'énergie disponible est donc un point crucial dans la mise en oeuvre des techniques de communication HBC entre capsules autonomes, et il est indispensable de concevoir des techniques réduisant au minimum les besoins en énergie de ces capsules.

Le problème de l'invention est, dans un tel contexte, de pouvoir déterminer le décalage existant entre les horloges de deux dispositifs indépendants, de manière à pouvoir mettre en oeuvre les actions correctrices appropriées.

Un autre problème réside dans le temps de transmission des impulsions d'un dispositif à l'autre.

En effet, comme on l'a indiqué plus haut, la transmission d'informations se fait par propagation d'impulsions au sein des tissus interstitiels du corps du patient, et cette propagation se fait dans un temps non négligeable. À ce temps de propagation doit être ajouté le temps de réponse de l'amplificateur faible bruit (amplificateur de type LNA, *Low Noise Amplifier)* nécessaire pour détecter une impulsion en entrée du dispositif récepteur.

Ces deux durées ajoutées conduisent à un "temps de transmission" dont il n'est la plupart du temps pas possible de faire abstraction, et qui s'ajoute à la désynchronisation propre des horloges.

L'invention propose à cet effet un procédé de quantification de la désynchronisation entre deux horloges d'un ensemble comprenant au moins deux dispositifs médicaux implantables actifs du type général divulgué par le US 2007/0088397 A1 précité, c'est-à-dire aptes à communiquer entre eux sans fil par voie intracorporelle au moyen de signaux constitués notamment de trains d'impulsions électriques modulées conduites par les tissus interstitiels du corps.

L'invention n'est toutefois pas limitée à ce mode de transmission des impulsions, et s'applique également au cas de signaux constitués d'autre types d'ondes conduites par les tissus interstitiels du corps, ou d'ondes acoustiques ou d'impulsions ou ondes magnétiques ou électromagnétiques.

L'ensemble comprend les éléments divulgués par le US2009/030484 A1 précité, correspondant au préambule de la revendication 1, à savoir : un premier dispositif comprenant une première horloge lente, des premiers moyens émetteurs/récepteurs desdits signaux, et un premier circuit de traitement numérique cadencé par la première horloge lente ; et un second dispositif comprenant une seconde horloge lente, des seconds moyens émetteurs/récepteurs desdits signaux, et un second circuit de traitement numérique cadencé par la seconde horloge lente. Pour quantifier par le second dispositif le défaut de synchronisme de la seconde horloge lente par rapport à la première horloge lente, le premier et/ou le second dispositif comprend une horloge rapide.

De façon caractéristique de l'invention, le procédé comprend les étapes suivantes :

a) par le second dispositif, sur une transition prédéterminée de la seconde horloge lente, émission à destination du premier dispositif d'un signal de requête de synchronisation ;
b) par l'horloge rapide du premier ou du second dispositif, comptage des impulsions de cette horloge rapide jusqu'à détection d'une transition prédéterminée de la première horloge lente ; puis
c) par le premier dispositif, émission à destination du second dispositif d'un signal de réponse à la requête de synchronisation ; et
d) par le second dispositif, sur réception du signal de réponse à la requête de synchronisation, calcul du décalage temporel correspondant audit défaut de synchronisme en fonction du résultat du comptage des impulsions de la première horloge rapide et/ou de ladite horloge rapide.

Dans un premier mode de réalisation, simplifié, où c'est le premier dispositif qui comprend une première horloge rapide :

- l'étape b) comprend le comptage des impulsions de cette première horloge rapide jusqu'à détection de ladite transition prédéterminée de la première horloge lente, et
- ledit signal de réponse à la requête de synchronisation émis à l'étape c) contient le résultat de ce comptage des impulsions de la première horloge rapide.

Dans un deuxième mode de réalisation, simplifié, où c'est le second dispositif qui comprend une seconde horloge rapide :

- l'étape a) comprend le comptage des impulsions de cette seconde horloge rapide jusqu'à réception à l'étape d) dudit signal de réponse à la requête de synchronisation.

Dans l'un ou l'autre de ces deux cas, le calcul du défaut de synchronisme peut être obtenu, abstraction faite du temps de propagation des impulsions électriques conduites entre les premier et second dispositifs, par application d'un relation de la forme :

$$OFFSET = Tclk - D,$$

OFFSET étant la valeur du décalage temporel correspondant audit défaut de synchronisme, Tclk étant la période des horloges lentes, et D étant la durée correspondant au comptage des impulsions de la première ou de la seconde horloge rapide.
Dans un troisième mode de réalisation, complet, où à la fois le premier dispositif comprend une première horloge rapide et le second dispositif comprend une seconde horloge rapide :

- l'étape a) comprend le début du comptage des impulsions de la seconde horloge rapide ;
- l'étape b) comprend le comptage des impulsions de la première horloge rapide jusqu'à détection de ladite transition prédéterminée de la première horloge lente,
- le signal de réponse à la requête de synchronisation émis à l'étape c) contient le résultat du comptage des impulsions de la première horloge rapide ; et
- l'étape d) comprend le calcul dudit décalage temporel en fonction des résultats à la fois du comptage des impulsions de la seconde horloge rapide et du comptage des impulsions de la première horloge rapide.

Dans ce denier cas, le calcul du défaut de synchronisme peut être obtenu, en tenant compte du temps de propagation des impulsions électriques conduites entre les premier et second dispositifs, par application d'un relation de la forme :

$$OFFSET = Tclk - [(D1+D2)/2],$$

OFFSET étant la valeur du décalage temporel correspondant audit défaut de synchronisme, Tclk étant la période des horloges lentes, D1 étant la durée correspondant au comptage des impulsions de la première horloge rapide, et D2 étant la durée correspondant au comptage des impulsions de la seconde horloge rapide.
Avantageusement, dans une variante de réalisation des divers modes de réalisation ci-dessus, les première et/ou seconde horloges rapides sont des horloges sélectivement activables et initialement désactivées, tandis que les première et seconde horloges lentes sont activées de façon continue, et dans le procédé :

- à l'étape a) il est prévu en outre une sous-étape d'activation de la seconde horloge rapide avant le début du comptage des impulsions, et à l'étape d) il est prévu en outre une sous-étape de désactivation de la seconde horloge rapide après réception du signal de réponse à la requête de synchronisation; et/ou
- à l'étape b) il est prévu en outre des sous-étapes d'activation de la première horloge rapide avant le début du comptage des impulsions, et de désactivation de celle-ci après détection de la transition prédéterminée de la première horloge lente.

Les fréquences des première et seconde horloges lentes sont préférentiellement égales et comprises entre 15 et 50 kHz, de préférence entre 30 et 35 kHz, et la fréquences des horloges rapides est de préférence au moins 10 fois la fréquence des horloges lentes. La précision des horloges lentes est avantageusement meilleure que 1 %.

On va maintenant décrire un exemple de mise en oeuvre de l'invention, en référence aux dessins annexés où les mêmes références numériques désignent d'une figure à l'autre des éléments identiques ou fonctionnellement semblables.

La Figure 1 illustre de façon schématique un ensemble de dispositifs médicaux comprenant notamment des capsules *leadless* implantées au sein du corps d'un patient.

La Figure 2 est un schéma par blocs fonctionnels montrant les différents étages constitutifs de deux dispositifs de l'ensemble de la Figure 1 et la manière dont ceux-ci communiquent.

La Figure 3 présente une série de chronogrammes destinés à expliquer le fonctionnement du procédé selon l'invention de détermination du degré de désynchronisation des deux dispositifs.

La Figure 4 présente, parallèlement, deux organigrammes correspondant aux différentes étapes mises en oeuvre respectivement au sein de chacun des deux dispositifs pour la mise en oeuvre du procédé de l'invention.

On va maintenant décrire un exemple de réalisation de l'invention.

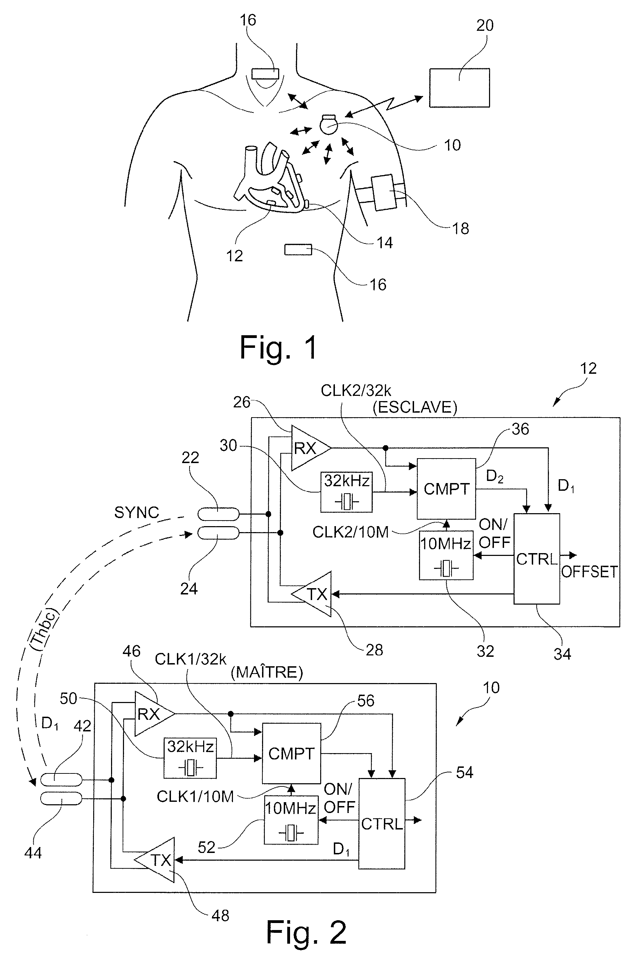

Sur la Figure 1 on a illustré un ensemble de dispositifs médicaux implantés au sein du corps d'un patient, communiquant entre eux sans fil par voie "HBC" *(Human Body Communication,* communication par voie intra-corporelle).

Le patient est équipé par exemple d'un implant 10 tel qu'un défibrillateur/stimulateur/resynchroniseur implanté, ou un défibrillateur de type sous-cutané, ou encore un enregistreur longue durée. Ce dispositif implanté 10 est le dispositif maître d'un réseau comportant une pluralité de dispositifs esclaves 12 à 18 avec lesquels il est susceptible d'entrer en communication par voie HBC. Le maître peut être également une capsule, sans nécessiter la présence d'un défibrillateur/stimulateur/resynchroniseur implanté sous-cutané relié à une ou plusieurs sondes, ou bien un défibrillateur de type sous-cutané.

Ces dispositifs peuvent notamment inclure des capsules intracardiaques 12 ou épicardiques 14 implantées directement sur le coeur du patient. De telles capsules sont par exemple décrites dans les US 2007/0088397 A1, WO 2007/047681 A2 et US 2006/0136004 A1, et sont fixées à la paroi cardiaque au moyen d'une vis d'ancrage saillante destinée à pénétrer dans le tissu cardiaque par vissage au site d'implantation. La vis peut être soit une vis passive, ne servant qu'à la fixation de la capsule, soit une vis active, servant à recueillir les signaux de dépolarisation se propageant dans les tissus du myocarde et/ou à délivrer des impulsions de stimulation au site d'implantation, de façon localisée.

L'ensemble peut également comprendre d'autres dispositifs 16 tels que capteurs de myopotentiels ou dispositifs de stimulation neurologique, et éventuellement un dispositif externe 18 disposé sur un brassard et pourvu d'électrodes en contact avec la peau. Le dispositif 10 peut également être utilisé en tant que passerelle avec le monde extérieur pour communiquer avec un périphérique externe 20 du type programmateur ou dispositif de télétransmission de données avec lequel il pourra communiquer notamment par télémétrie RF dans la bande MICS (*Medical Implants Communication System*) 402-405 MHz, ou les bandes MEDS 401-402 MHz et 405-406 MHz, ou encore les bandes banalisées publiques ISM (Industriel, Scientifique et Médical) 863-870 MHz, 902-928 MHz et 2,4 GHz utilisées par les dispositifs médicaux.

Chacun des dispositifs 10 à 18 est muni d'au moins un couple d'électrodes qui se trouvent en contact direct avec les tissus du corps pour les dispositifs implantés, ou en contact avec la peau pour le dispositif externe 18, ou relié à des sondes, ou comportant des électrodes sur le connecteur ou le boîtier pour le dispositif 10.

L'un des dispositifs, par exemple le stimulateur/défibrillateur 10, peut jouer un rôle de concentrateur ou de dispositif maître dans une architecture de réseau sans fil en étoile, dont les diverses capsules *leadless* 12 à 18 seront des dispositifs esclaves - ceci pour une communication du stimulateur/défibrillateur vers la capsule ; pour une communication dans l'autre sens, les rôles seront inversés.

La nature de la communication entre les différents dispositifs, en réseau ou non, et le type de réseau considéré, ne font cependant pas partie de l'invention, qui concerne seulement la communication initiale entre deux dispositifs implantés quelconques.

On prendra l'exemple de la communication entre le stimulateur/défibrillateur 10 en tant que dispositif maître et une capsule intracardiaque 12 en tant que dispositif esclave, mais cet exemple est purement illustratif et l'invention peut être appliquée à la communication entre deux quelconques des dispositifs 10 à 20 présentés plus haut, et ceci indifféremment dans un sens ou dans l'autre (c'est-à-dire que les rôles de maître et d'esclave peuvent être inversés).

Sur la Figure 2 on a illustré de façon schématique les différents circuits internes du stimulateur/défibrillateur 10 et de la capsule *leadless* 12, nécessaires à la mise en oeuvre de l'invention. On ne décrira que les éléments constitutifs 22 à 36 de la capsule *leadless* 12, sachant que le stimulateur/défibrillateur 10 contient des éléments semblables 42 à 56, correspondant respectivement aux éléments 22 à 36 décrits dans le cadre de la capsule.

La capsule 12 comporte deux électrodes 22, 24 en contact direct avec les tissus du corps, permettant l'émission et la réception de signaux constitués d'impulsions conduites par les tissus interstitiels du corps situés entre les deux dispositifs 10 et 12, et servant aussi à la détection et à la stimulation cardiaque. Pour la capsule, l'une des électrodes 22 ou 24 peut être constituée par la vis d'ancrage dans le tissu du coeur, ou par une ou des électrodes sur le boîtier ou reliées au boîtier. Le couple d'électrodes 22, 24 est relié à un circuit 26 de détection des potentiels recueillis entre les électrodes, et à un circuit émetteur 28 pour la production des impulsions HBC pour la communication avec le dispositif distant 10.

Les électrodes 22, 24 peuvent être également reliées à un circuit générateur d'impulsions de stimulation (non représenté) si la capsule 12 incorpore cette fonction et/ou à un circuit de recueil des potentiels de dépolarisation cardiaque spontanés ou stimulés (ces derniers potentiels sont à distinguer des potentiels correspondant aux signaux HBC, qui sont émis à un niveau d'énergie très inférieur à celui des potentiels cardiaques et des myopotentiels de l'organisme). En d'autres termes, les électrodes 22, 24 assurent plusieurs fonctions, à savoir le recueil des potentiels cardiaques et/ou la stimulation (le cas échéant), et en tout état de cause l'émission/réception pour la communication HBC. Ces circuits ne participant pas à l'invention, ils n'ont pas été illustrés sur la Figure 2.

La capsule peut également être pourvue d'un capteur tel qu'un capteur d'accélération, de pression, un capteur hémodynamique, de température, de saturation en oxygène, etc. Elle est alimentée par un circuit de récupération d'énergie ou une petite batterie.

De façon caractéristique, le dispositif 12 est pourvu de deux horloges distinctes, à savoir une horloge lente 30 et une horloge rapide 32. Les termes de "rapide" et "lente" doivent être entendus dans leur sens relatif, c'est-à-dire que la fréquence de l'horloge 32 est supérieure à celle de l'horloge 30, avec une différence d'au moins un ordre de grandeur (dix fois).

L'horloge lente 30 produit des impulsions CLK2/32k à une fréquence par exemple de 32 768 Hz, tandis que l'horloge rapide 32 produit des impulsions CLK2/10M à une fréquence de 9,83040 MHz, c'est-à-dire trois cents fois supérieure à celle de l'horloge lente 30. Les valeurs précises de ces fréquences ne sont données qu'à titre d'exemple, et ont été choisies pour disposer de fréquences présentant entre elles un rapport simple, facile à générer par division de fréquence. Pour la simplicité de l'exposé, on parlera simplement de fréquence à "32 kHz" et "10 MHz", la valeur précise choisie n'étant pas en elle-même importante pour la mise en oeuvre de l'invention. Par ailleurs, les impulsions correspondant à ces fréquences à 32 kHz et 10 MHz générées au sein du dispositif 12 seront désignées CLK2/32k et CLK2/10M, respectivement ; celles générées au sein du dispositif 10 seront désignées CLK1/32k et CLK1/10M, respectivement.

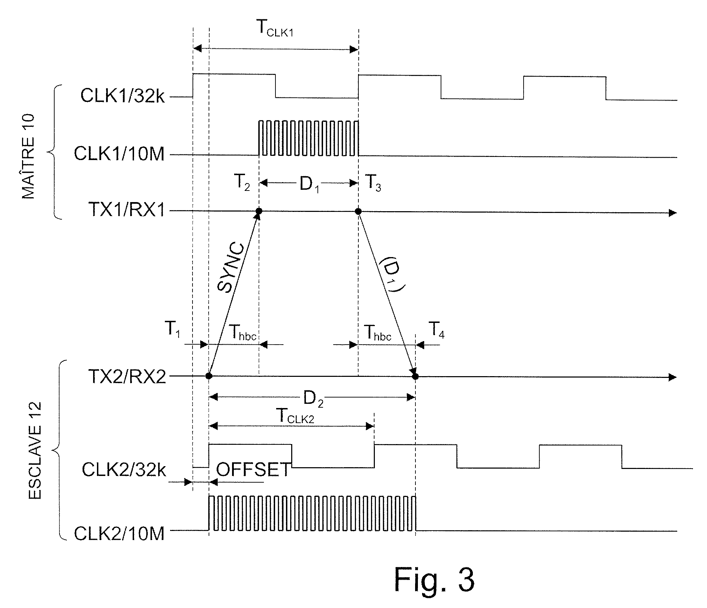

Les signaux d'horloge lente et rapide sont générés sous forme de signaux carrés de rapport cyclique 50 %, comme on peut le voir sur les chronogrammes de la Figure 3. Les périodes correspondantes sont donc de l'ordre de 30 μs pour l'horloge lente et 0,1 μs pour l'horloge rapide.

L'horloge lente 30 génère les impulsions de façon continue, en permanence, et ces impulsions servent au séquencement d'un circuit central à microcontrôleur (non représenté) incluant l'ensemble de l'électronique permettant de piloter les diverses fonctions de la capsule, la mémorisation des signaux recueillis, etc. L'horloge lente 30 génère ainsi les signaux d'horloge nécessaires notamment (i) à l'analyse des signaux de dépolarisation recueillis par les électrodes, à la génération des impulsions de stimulateur, etc. et (ii) à la communication HBC avec les autres dispositifs implantés, notamment avec le dispositif maître constitué par le stimulateur/défibrillateur 10.

L'horloge rapide 32, en revanche, n'est activée que de façon temporaire, dans les conditions que l'on décrira plus bas. L'état normal de cette horloge est un état désactivé, et l'activation en est contrôlée par un circuit 34 commandé à partir de données D1 reçues du dispositif maître 10 et de données D2 élaborées en interne par le dispositif 12, notamment par un circuit de comptage 36 comptant les impulsions CLK2/10M de l'horloge rapide 32, ce circuit 36 étant lui-même contrôlé (i) par l'horloge lente 30 et (ii) par des signaux reçus du dispositif maître 10 par communication HBC. On a choisi pour l'horloge 30 une valeur de fréquence relativement basse de manière à limiter la consommation et préserver l'autonomie énergétique de l'implant. C'est pour cette raison que l'horloge rapide 32 n'est activée que lorsque cela est nécessaire, car sa consommation, très supérieure, serait incompatible avec un fonctionnement permanent de l'implant, dont les ressources énergétiques sont très réduites (circuit de récupération d'énergie pour la capsule *leadless,* notamment, ou batterie à très longue durée de vie (10 ans) pour le stimulateur/défibrillateur 10).

Le dispositif maître constitué par le stimulateur/défibrillateur 10 comporte les mêmes éléments 42 à 56 que les éléments 22 à 36 du dispositif esclave constitué par la capsule *leadless* 12, comme on l'a indiqué plus haut.

Ainsi, chacun des dispositifs 10 et 12 est pourvu de sa propre horloge lente 30 ou 50, respectivement, qui assure le séquencement des divers circuits numériques de chacun des dispositifs. Ces deux horloges étant physiquement indépendante, elles présentent un certain degré de désynchronisation, ou *offset* temporel.

Il s'agit de pouvoir calculer ce décalage afin de pouvoir le prendre en compte dans la suite du protocole, de façon notamment à ce que les fenêtres de réception et d'émission des dispositifs maître et esclave coïncident le plus précisément possible pour la communication HBC.

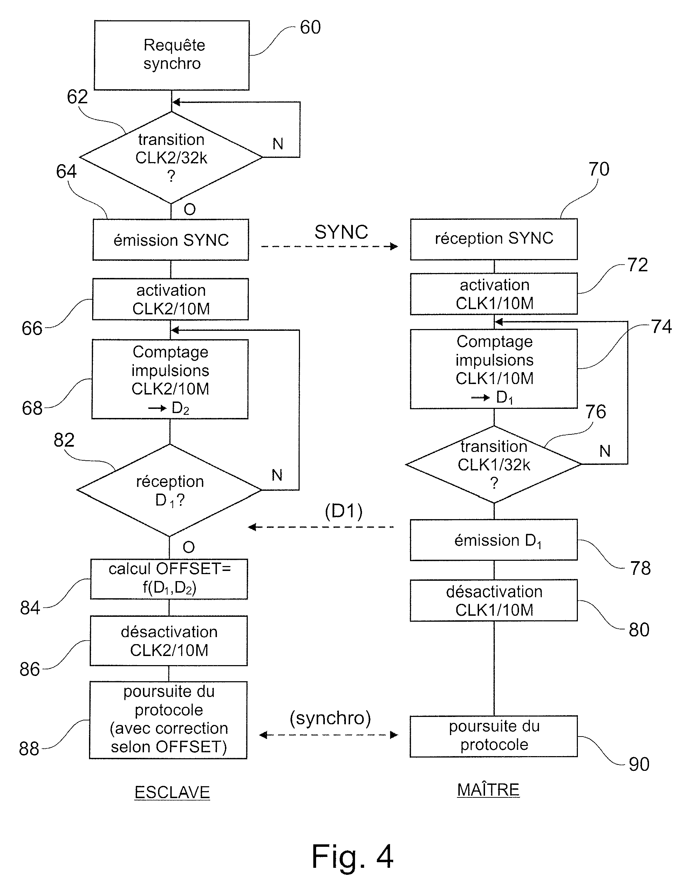

On va maintenant décrire la manière détaillée dont est opérée la détermination de ce décalage temporel au sein du dispositif esclave 12, en référence aux Figures 3 et 4.

Sur les chronogrammes de la Figure 3, ce décalage temporel indiqué par le paramètre OFFSET (à déterminer) est celui présent entre les signaux des horloges lente CLK1/32k et CLK2/32k.

Le dispositif esclave 12 qui souhaite se synchroniser attend une transition prédéterminée (par exemple le front montant) de son horloge lente CLK2/32k pour émettre, à un instant T1, une impulsion SYNC en direction du dispositif maître 10 (étapes 60, 62, 64 sur l'organigramme de la Figure 4).

Le dispositif maître 10 reçoit cette impulsion de requête de synchronisation (étape 70) à l'instant T2, avec par rapport à l'instant d'émission de cette impulsion T1 un retard de durée (T2-T1) = Thbc correspondant au temps de transmission de l'impulsion au travers des tissus interstitiels du corps depuis le dispositif esclave 12 jusqu'au dispositif maître 10.

Sur réception de l'impulsion SYNC, le dispositif maître 10 active son horloge rapide 52 (étape 72), par une action du circuit de contrôle 54 après détection de l'impulsion SYNC par le circuit de réception 46. Le dispositif maître 10 va alors compter le nombre d'impulsions CLK1/10M générées par cette horloge rapide 52, au moyen du circuit de comptage 56 (étape 74).

Parallèlement, dès l'émission de l'impulsion SYNC, le dispositif esclave 12 active son horloge rapide 32 (étape 66) et commence à compter les impulsions CLK2/10M de cette horloge dans son circuit de comptage 36 (étape 68).

Du côté du dispositif maître 10, le comptage des impulsions CLK1/10M de l'horloge rapide se poursuit jusqu'à détection, à l'instant T3, d'une transition prédéterminée (par exemple un front montant) du signal CLK1/32k de l'horloge lente 50. Le nombre d'impulsions comptées donne une valeur numérique D1 représentative de la durée comprise entre les instants T2 et T3. Avec une horloge rapide CLK1/32k cadencée à 10 MHz, la correspondance est de 0,1 $\mu$s par impulsion d'horloge comptée.

Cette valeur D1 est transmise au dispositif esclave 12 au sein d'un message de réponse (étape 78). Puis l'horloge rapide 52 est désactivée (étape 80) pour limiter la consommation du dispositif, l'horloge lente 50 continuant en revanche à produire les impulsions CLK1/32k nécessaires au séquencement des divers circuits numériques du dispositif maître 10, notamment pour permettre la poursuite du protocole d'échange de données avec le dispositif esclave 12.

Du côté du dispositif esclave 12, le message en retour contenant la valeur D1 est reçu à l'instant T4, décalé de l'instant T3 d'une durée correspondant au temps de transmission Thbc du dispositif maître 10 vers le dispositif esclave 12, avec un retard (T4-T3) = Thbc (on suppose que le temps de transmission est le même dans un sens et dans l'autre).

On notera que le circuit 26 de détection des dispositifs esclaves n'est mis en route que dans la fenêtre de réception déclenchée par l'horloge rapide CLK2/10M de l'esclave. La durée de cette fenêtre est d'un nombre de périodes supérieur ou égal à l'une des périodes de CLK2/10M.

Sur réception de ce message en retour (étape 82) le dispositif esclave va calculer la valeur du décalage OFFSET à partir de la valeur D1 reçue et de la valeur D2 = (T4-T1) calculée en interne et correspondant au nombre d'impulsions CLK2/10M de l'horloge rapide 32 comptées par le circuit de comptage 36 (étape 84).

L'horloge rapide 32 est alors désactivée (étape 86) pour limiter la consommation, et le protocole d'échange de signaux peut être alors poursuivi (étape 88) pour permettre l'échange de signaux avec le dispositif maître 10 (étape 90). Cette communication peut être établie en utilisant le paramètre OFFSET pour appliquer ensuite une correction temporelle correspondante à la datation des signaux reçus ou émis (synchronisation logicielle).

La valeur du décalage OFFSET est calculée de la manière suivante.

Si l'on suppose que le temps de transmission Thbc est identique dans les deux sens de communication, on a :

$$T4 - T1 = 2.Thbc + D1 = D2$$

Connaissant D2 et D1 (D2 compté en interne par le dispositif esclave 12 et D1 compté à distance par le dispositif maître 10 et transmis au dispositif esclave 12), on en déduit :

$$Thbc = (D2 - D1) / 2.$$

Les oscillateurs à quartz utilisés pour ces horloges présentent une précision typique de 50 ppm, pour une

7

fréquence de 32768 Hz. Cette précision est suffisante, à l'échelle de la transmission d'un paquet de données en HBC, qui peut tolérer un défaut de synchronisme d'une période de l'horloge rapide, du fait de la tolérance de 50 ppm des quartz. De ce fait, si l'on désigne TCLK1 et TCLK2 les périodes des horloges lentes respectives 30 et 50 des dispositifs esclave 12 et maître 10, on peut considérer que Tclk1 = Tclk2 = Tclk. Il vient :

$$T3 - T1 = Tclk - OFFSET = D1 + Thbc,$$

d'où :

$$OFFSET = Tclk - D1 - Thbc = Tclk - (D1 + D2) / 2$$

Le décalage OFFSET ayant été ainsi calculé, on pourra déterminer et ajuster les fenêtres de communication avec le dispositif maître de manière à obtenir la synchronisation recherchée pendant la transmission du paquet de données.

La prise en compte du temps de propagation Thbc est facultative, et dépend de la précision recherchée.

Ce temps de propagation peut être évalué de façon très approximative à environ 500 ns, de sorte que si l'horloge rapide opère à une fréquence de 1 MHz au lieu de 10 MHz, ce temps de propagation peut être négligé dans la mesure où il sera pratiquement sans incidence sur le résultat du calcul du décalage OFFSET.

Dans ce cas, on pourra considérer que les instants T1 et T2 sont confondus, de même que les instants T3 et T4, et que de ce fait D1 = D2. Il n'est alors plus nécessaire de mesurer D2 (on peut faire abstraction de l'étape 68), et l'on a directement :

$$OFFSET = Tclk - D1$$

Dans la mesure où il n'est plus nécessaire de mesurer D2, le dispositif esclave 12 peut être dépourvue d'horloge rapide, ce qui permet de simplifier la conception, de diminuer la taille et de réduire la consommation du dispositif esclave.

En variante, puisque D1 = D2, si le dispositif esclave 12 est pourvu d'une horloge rapide on peut évaluer D1 au moyen de cette horloge rapide, qui donnera la valeur de D2, égale à D1. Cette solution requiert certes la présence d'une horloge rapide dans le dispositif esclave 12, mais en revanche elle simplifie le protocole d'échange des signaux avec le dispositif maître 10 : en effet, il suffit au dispositif esclave 12 de détecter la réception d'un marqueur venant du dispositif maître 10 et correspondant à la détection de la transition de l'horloge lente de celui-ci, sans qu'il soit nécessaire de transmettre depuis ce dernier une valeur numérisée (la valeur de D1).

De façon générale, un maître 10 pourra resynchroniser concurremment un ensemble d'esclaves en émettant une impulsion de synchronisation sur un front montant de CLK1/32K qui sera détectée par chaque esclave 14 ou 12 par exemple.

Chaque esclave débute le comptage des impulsions CLK2/10M à la détection de cette impulsion de synchronisation, jusqu'au prochain front montant de CLK2/32K.

Un maître 10 pourra maintenir des esclaves 12 ou 14 synchronisés en émettant régulièrement une impulsion de synchronisation par exemple toutes les 2 ms.

L'esclave 12 ou 14 pourra utiliser cette impulsion de synchronisation comme horloge interne. L'esclave n'aura pas besoin d'un oscillateur à quartz comme horloge interne. De là résulte un gain en consommation et en encombrement.

Lorsque l'esclave aura besoin d'exécuter des opérations plus rapides, il pourra mettre en route une horloge RC rapide qu'il interrompra dès l'achèvement des traitements.

La stimulation et la décharge des capacités de stimulation du maître et des esclaves seront synchrones de l'horloge de 2 ms du maître, ce qui leur permet de servir aussi d'impulsion de synchronisation.

## Revendications

1.  Un procédé de quantification de la désynchronisation entre deux horloges d'un ensemble comportant au moins deux dispositifs médicaux implantables actifs (10, 12) aptes à communiquer entre eux sans fil par voie intracorporelle au moyen de signaux constitués de trains d'impulsions ou d'ondes conduits par les tissus interstitiels du corps, avec :

    - un premier dispositif (10) comprenant une première horloge lente (30), des premiers moyens émetteurs/récepteurs (26, 28) desdits signaux, et un premier circuit de traitement numérique cadencé par la première horloge lente ; et
- un second dispositif (12) comprenant une seconde horloge lente (50), des seconds moyens émetteurs/récepteurs (46, 48) desdits signaux, et un second circuit de traitement numérique cadencé par la seconde horloge lente,

et dans lequel, pour quantifier par le second dispositif le défaut de synchronisme de la seconde horloge lente par rapport à la première horloge lente, le premier (10) et/ou le second dispositif (12) comprend une horloge rapide (32, 52),
ce procédé étant **caractérisé en ce qu'**il comprend les étapes suivantes :

a) par le second dispositif, sur une transition prédéterminée de la seconde horloge lente, émission (60) à destination du premier dispositif d'un signal de requête de synchronisation (SYNC) ;
b) par l'horloge rapide du premier ou du second dispositif, comptage (68, 74) des impulsions de cette horloge rapide jusqu'à détection (76) d'une transition prédéterminée de la première horloge lente ; puis
c) par le premier dispositif, émission (78) à destination du second dispositif d'un signal de réponse à la requête de synchronisation ; et
d) par le second dispositif, sur réception (82) du signal de réponse à la requête de synchronisation, calcul (84) du décalage temporel (OFFSET) correspondant audit défaut de synchronisme en fonction du résultat (D1, D2) du comptage (68, 74) des impulsions de ladite horloge rapide.

2. Le procédé de la revendication 1, dans lequel, le premier dispositif comprenant une première horloge rapide (32) :

- l'étape b) comprend le comptage (74) des impulsions de cette première horloge rapide jusqu'à détection de ladite transition prédéterminée de la première horloge lente, et
- ledit signal de réponse à la requête de synchronisation émis à l'étape c) contient le résultat (D1) de ce comptage des impulsions de la première horloge rapide.

3. Le procédé de la revendication 1, dans lequel, le second dispositif comprenant une seconde horloge rapide (52) :

- l'étape a) comprend le comptage (68) des impulsions de cette seconde horloge rapide jusqu'à réception à l'étape d) dudit signal de réponse à la requête de synchronisation.

4. Le procédé de la revendication 2 ou 3, dans lequel le calcul dudit défaut de synchronisme est obtenu, abstraction faite du temps de propagation (Thbc) des impulsions électriques conduites entre les premier et second dispositifs, par application d'un relation de la forme :

$$OFFSET = Tclk - D,$$

OFFSET étant la valeur du décalage temporel correspondant audit défaut de synchronisme,
Tclk étant la période des horloges lentes, et
D étant la durée (D1, D2) correspondant au comptage des impulsions de la première (D1) ou de la seconde (D2) horloge rapide.

5. Le procédé de la revendication 1, dans lequel, le premier dispositif comprenant une première horloge rapide (32) et le second dispositif (20) comprenant une seconde horloge rapide (52) :

- l'étape a) comprend le début du comptage (68) des impulsions de la seconde horloge rapide ;
- l'étape b) comprend le comptage (74) des impulsions de la première horloge rapide jusqu'à détection de ladite transition prédéterminée de la première horloge lente,
- le signal de réponse à la requête de synchronisation émis à l'étape c) contient le résultat (D1) du comptage des impulsions de la première horloge rapide ; et
- l'étape d) comprend le calcul (84) dudit décalage temporel (OFFSET) en fonction des résultats (D1, D2) à la fois du comptage des impulsions de la seconde horloge rapide et du comptage des impulsions de la première horloge rapide.

**6.** Le procédé de la revendication 5, dans lequel le calcul dudit défaut de synchronisme est obtenu, en tenant compte du temps de propagation des impulsions électriques conduites entre les premier et second dispositifs, par application d'un relation de la forme :

$$OFFSET = Tclk - [(D1+D2)/2],$$

OFFSET étant la valeur du décalage temporel correspondant audit défaut de synchronisme,
Tclk étant la période des horloges lentes,
D1 étant la durée correspondant au comptage des impulsions de la première horloge rapide, et
D2 étant la durée correspondant au comptage des impulsions de la seconde horloge rapide.

**7.** Le procédé de la revendication 1, dans lequel :

- les première et/ou seconde horloges rapides (32, 52) sont des horloges sélectivement activables et initialement désactivées, tandis que les première et seconde horloges lentes (30, 50) sont activées de façon continue ;

et dans lequel :

- à l'étape a) il est prévu en outre une sous-étape d'activation (66) de la seconde horloge rapide avant le début du comptage des impulsions, et à l'étape d) il est prévu en outre une sous-étape de désactivation (86) de la seconde horloge rapide après réception du signal de réponse à la requête de synchronisation; et/ou
- à l'étape b) il est prévu en outre des sous-étapes d'activation (72) de la première horloge rapide avant le début du comptage des impulsions, et de désactivation (80) de celle-ci après détection de la transition prédéterminée de la première horloge lente.

**8.** Le procédé de la revendication 1, dans lequel les fréquences des première et seconde horloges lentes sont égales et comprises entre 15 et 50 kHz, de préférence entre 30 et 35 kHz.

**9.** Le procédé de la revendication 1, dans lequel la fréquences de l'(des) horloge(s) rapide(s) est au moins 10 fois la fréquence des horloges lentes.

**10.** Le procédé de la revendication 1, dans lequel la précision des horloges lentes est meilleure que 1 %.

**Patentansprüche**

**1.** Verfahren zur Quantifizierung der Desynchronisation zwischen zwei Uhren einer Einheit, umfassend mindestens zwei aktive implantierbare medizinische Vorrichtungen (10, 12), die geeignet sind, drahtlos untereinander auf intrakorporalem Weg mit Hilfe von Signalen zu kommunizieren, die von Impuls- oder Wellenfolgen gebildet sind, die von den interstitiellen Geweben des Körpers geleitet werden, mit:

- einer ersten Vorrichtung (10), umfassend eine erste langsame Uhr (30), erste Sende-/Empfangsmittel (26, 28) der Signale, und eine erste digitale Verarbeitungsschaltung, die durch die erste langsame Uhr getaktet ist; und
- einer zweiten Vorrichtung (12), umfassend eine zweite langsame Uhr (50), zweite Sende-/Empfangsmittel (46, 48) der Signale, und eine zweite digitale Verarbeitungsschaltung, die durch die zweite langsame Uhr getaktet ist,

und bei dem, um durch die zweite Vorrichtung den Synchronfehler der zweiten langsamen Uhr in Bezug zur ersten langsamen Uhr zu quantifizieren, die erste (10) und/oder die zweite Vorrichtung (12) eine schnelle Uhr (32, 52) umfasst bzw. umfassen, wobei dieses Verfahren **dadurch gekennzeichnet ist, dass** es die folgenden Schritte umfasst:

a) durch die zweite Vorrichtung auf einem vorbestimmten Übergang der zweiten langsamen Uhr Senden (60) in Richtung der ersten Vorrichtung eines Synchronisations-Anfragesignals (SYNC);
b) durch die schnelle Uhr der ersten oder der zweiten Vorrichtung Zählen (68, 74) der Impulse dieser schnellen Uhr bis zur Erfassung (76) eines vorbestimmten Übergangs der ersten langsamen Uhr; dann

c) durch die erste Vorrichtung Senden (78) in Richtung der zweiten Vorrichtung eines Antwortsignals auf die Synchronisationsanfrage; und

d) durch die zweite Vorrichtung bei Empfang (82) des Antwortsignals auf die Synchronisationsanfrage Berechnung (84) des Zeitversatzes (OFFSET) entsprechend dem Synchronfehler in Abhängigkeit von dem Resultat (D1, D2) des Zählens (68, 74) der Impulse der schnellen Uhr.

2. Verfahren nach Anspruch 1, bei dem, wobei die erste Vorrichtung eine erste schnelle Uhr (32) umfasst:

- der Schritt b) das Zählen (74) der Impulse dieser ersten schnellen Uhr bis zur Erfassung des vorbestimmten Übergangs der ersten langsamen Uhr umfasst, und
- das Antwortsignal auf die in Schritt c) gesandte Synchronisationsanfrage das Resultat (D1) dieses Zählens der Impulse der ersten schnellen Uhr enthält.

3. Verfahren nach Anspruch 1, bei dem, wobei die zweite Vorrichtung eine zweite schnelle Uhr (52) umfasst:

- der Schritt a) das Zählen (68) der Impulse dieser zweiten schnellen Uhr bis zum Empfang des Antwortsignals auf die Synchronisationsanfrage in Schritt d) umfasst.

4. Verfahren nach Anspruch 2 oder 3, bei dem die Berechnung des Synchronfehlers, abgesehen von der Ausbreitungszeit (Thbc) der elektrischen Impulse, die zwischen der ersten und der zweiten Vorrichtung geleitet werden, erhalten wird durch Anwendung eines Verhältnisses folgender Form:

$$OFFSET = Tclk - D,$$

wobei OFFSET der Wert des Zeitversatzes entsprechend dem Synchronfehler ist,
Tclk die Periode der langsamen Uhren ist, und
D die Dauer (D1, D2) entsprechend dem Zählen der Impulse der ersten (D1) und der zweiten (D2) schnellen Uhr ist.

5. Verfahren nach Anspruch 1, bei dem, wobei die erste Vorrichtung eine erste schnelle Uhr (32) umfasst, und die zweite Vorrichtung (20) eine zweite schnelle Uhr (52) umfasst,

- der Schritt a) den Beginn des Zählens (68) der Impulse der zweiten schnellen Uhr umfasst;
- der Schritt b) das Zählen (74) der Impulse der ersten schnellen Uhr bis zur Erfassung des vorbestimmten Übergangs der ersten langsamen Uhr umfasst,
- das Antwortsignal auf die Synchronisationsanfrage, das in Schritt c) gesandt wird, das Resultat (D1) des Zählens der Impulse der ersten schnellen Uhr enthält; und
- der Schritt d) die Berechnung (84) des Zeitversatzes (OFFSET) in Abhängigkeit von den Resultaten (D1, D2) sowohl des Zählens der Impulse der zweiten schnellen Uhr als auch des Zählens der Impulse der ersten schnellen Uhr umfasst.

6. Verfahren nach Anspruch 5, bei dem die Berechnung des Synchronfehlers erhalten wird, wobei die Ausbreitungszeit der elektrischen Impulse, die zwischen den ersten und zweiten Vorrichtungen geleitet werden, in Betracht gezogen wird, durch Anwendung eines Verhältnisses der folgenden Form:

$$OFFSET = Tclk - [(D1+D2)/2],$$

wobei OFFSET der Wert des Zeitversatzes entsprechend dem Synchronfehler ist,
Tclk die Periode der langsamen Uhren ist,
D1 die Dauer entsprechend dem Zählen der Impulse der ersten schnellen Uhr ist, und
D2 die Dauer entsprechend dem Zählen der Impulse der zweiten schnellen Uhr ist.

7. Verfahren nach Anspruch 1, bei dem:

- die ersten und/oder zweiten schnellen Uhren (32, 52) selektiv aktivierbare und anfänglich deaktivierte Uhren sind, während die ersten und zweiten langsamen Uhren (30, 50) kontinuierlich aktiviert sind;

und bei dem:

- in Schritt a) ferner ein Unterschritt (66) der Aktivierung der zweiten schnellen Uhr vor dem Beginn des Zählens der Impulse vorgesehen ist, und in Schritt d) ferner ein Unterschritt (86) der Deaktivierung der zweiten schnellen Uhr nach Empfang des Antwortsignals auf die Synchronisationsanfrage vorgesehen ist; und/oder
- in Schritt b) ferner Unterschritte der Aktivierung (72) der ersten schnellen Uhr vor dem Beginn des Zählens der Impulse und der Deaktivierung (80) derselben nach Erfassung des vorbestimmten Übergangs der ersten langsamen Uhr vorgesehen ist.

8. Verfahren nach Anspruch 1, bei dem die Frequenzen der ersten und der zweiten langsamen Uhr gleich sind und zwischen 15 und 50 kHz, vorzugsweise zwischen 30 und 35 kHz betragen.

9. Verfahren nach Anspruch 1, bei dem die Frequenz der schnellen Uhr(en) mindestens 10-mal die Frequenz der langsamen Uhren beträgt.

10. Verfahren nach Anspruch 1, bei dem die Genauigkeit der langsamen Uhren besser als 1% ist.

**Claims**

1. A method for quantifying the desynchronization between two clocks of a unit including at least two active implantable medical devices (10, 12) adapted to wirelessly and intracorporeally communicate with each other by means of signals consisted of trains of pulses or waves conducted by the interstitial tissues of the body, with:

- a first device (10) comprising a first slow clock (30), first means (26, 28) for transmitting / receiving said signals, and a first digital processing circuit clocked by the first slow clock; and
- a second device (12) comprising a second slow clock (50), second means (46, 48) for transmitting / receiving said signals, and a second digital processing circuit clocked by the second slow clock, and wherein, for quantifying by the second device the failure of synchronism of the second slow clock with respect to the first slow clock, the first (10) and/or the second (12) device comprises a fast clock (32, 52),

said method being **characterized in that** it comprises the following steps:

a) by the second device, at a predetermined transition of the second slow clock, transmitting (60) to the first device a synchronization request signal (SYNC);
b) by the fast clock of the first or the second device, counting (68, 74) the pulses of this fast clock until detection (76) of a predetermined transition of the first slow clock; then
c) by the first device, transmitting (78) to the second device a signal of response to the synchronization request; and
d) by the second device, upon reception (82) of the signal of response to the synchronization request, calculating (84) the time offset (OFFSET) corresponding to said synchronization failure as a function of the result (D1, D2) of the counting (68, 74) of said fast clock pulses.

2. The method of claim 1, wherein, the first device comprising a first fast clock (32) :

- step b) comprises counting (74) the pulses of this first fast clock until detection of said predetermined transition of the first slow clock, and
- said signal of response to the synchronization request transmitted at step c) contains the results (D1) of this counting of the first fast clock pulses.

3. The method of claim 1, wherein, the second device comprising a second fast clock (52):

- step a) comprises counting (68) the pulses of this second fast clock until reception, at step d), of said signal of response to the synchronization request.

4. The method of claim 2 or 3, wherein the calculation of said synchronism failure is obtained, leaving aside the time of propagation (Thbc) of the electric pulses conducted between the first and second devices, by application of a relation of the form:

$$OFFSET = Tclk - D,$$

OFFSET being the value of the time offset corresponding to said synchronism failure,
Tclk being the period of the slow clocks, and
D being the duration (D1, D2) corresponding to the counting of the pulses of the first (D1) or the second (D2) fast clock.

5. The method of claim 1, wherein, the first device comprising a first fast clock (32) and the second device (20) comprising a second fast clock (52):

    - step a) comprises beginning counting (68) the pulses of the second fast clock ;
    - step b) comprises counting (74) the pulses of the first fast clock until detection of said predetermined transition of the first slow clock,
    - the signal of response to the synchronization request transmitted at step c) contains the result (D1) of the counting of the first fast clock pulses; and
    - step d) comprises calculating (84) said time offset (OFFSET) as a function of the results (D1, D2) both of the counting of the second fast clock pulses and the counting of the first fast clock pulses.

6. The method of claim 5, wherein the calculation of said synchronism failure is obtained, taking into account the time of propagation of the electric pulses conducted between the first and second devices, by application of a relation of the form:

$$OFFSET = Tclk - [(D1 + D2)/2],$$

OFFSET being the value of the time offset corresponding to said synchronism failure,
Tclk being the period of the slow clocks,
D1 being the duration corresponding to the counting of the first fast clock pulses, and
D2 being the duration corresponding to the counting of the second fast clock pulses.

7. The method of claim 1, wherein:

    - the first and/or second fast clocks (32, 52) are selectively activatable and initially deactivated clocks, whereas the first and second slow clocks (30, 50) are continuously activated;

    and wherein:

    - at step a) it is further provided a sub-step of activating (66) the second fast clock before beginning the pulse counting, and at step d) it is further provided a sub-step of deactivating (86) the second fast clock after reception of the signal of response to the synchronization request; and/or
    - at step b) it is further provided sub-steps of activating (72) the first fast clock before beginning the pulse counting, and of deactivating (80) the latter after detection of the predetermined transition of the first slow clock.

8. The method of claim 1, wherein the frequencies of the first and second slow clocks are equal to each other and comprised between 15 and 50 kHz, preferably between 30 and 35 kHz.

9. The method of claim 1, wherein the frequency of the fast clock(s) is at least 10 times the frequency of the slow clocks.

10. The method of claim 1, wherein the accuracy of the slow clocks is better than 1 %.

EP 2 486 953 B1

Fig. 1

Fig. 2

Fig. 3

Fig. 4

**EP 2 486 953 B1**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 20070088397 A1
- WO 2007047681 A2
- US 20060136004 A1
- US 5411535 A
- US 4987897 A
- US 2009030484 A1
- US 20070255330 A1
- US 2050197680 A1
- US 20070088394 A1
- WO 2011093916 A